# EUROPEAN PATENT SPECIFICATION

(11) **EP 4 025 283 B1**
(45) Date of publication and mention of the grant of the patent: **06.11.2024**
(21) Application number: 20861504.7
(22) Date of filing: 03.09.2020
(51) Int. Cl.: A61M 16/04

(54) **PHARYNGEAL TUBE FOR ESTABLISHING A PATENT AIRWAY**
PHARYNGEALTUBUS ZUR HERSTELLUNG EINES OFFENEN ATEMWEGS
TUBE PHARYNGÉ POUR L'ÉTABLISSEMENT D'UNE VOIE RESPIRATOIRE DÉGAGÉE

(30) Priority: 03.09.2019 US 201962895295 P; 01.04.2020 CN 202010249473
(43) Date of publication of application: 13.07.2022
(73) Proprietor: Board of Regents of the University of Texas System, Austin, TX 78701 (US)
(72) Inventor: JIANG, Yandong, Bellaire, TX 77401 (US)
(74) Representative: Murgitroyd & Company
(86) International application number: PCT/US2020/049154
(87) International publication number: WO 2021/046188

(56) References cited:
- WO-A1-2007/061076
- WO-A1-2007/061076
- WO-A1-2016/034572
- DE-A1- 3 303 582
- US-A- 3 841 319
- US-A- 4 300 550
- US-A- 4 449 526
- US-A- 5 606 968
- US-A1- 2007 163 596
- US-A1- 2007 163 596
- US-A1- 2008 078 402
- US-A1- 2009 125 002
- US-A1- 2012 080 037
- US-A1- 2014 216 449
- US-A1- 2018 021 606
- US-B2- 8 955 518

## Description

### RELATED APPLICATIONS

This application claims the benefit of U.S. Provisional Patent Application No. 62/895,295 filed on Sept. 3, 2019 and CN Application No. 202010249473.0 filed on April 1, 2020.

### Background

When a patient has been administered general anesthesia for a surgical procedure or for emergency airway access in cases requiring ventilatory support, it is common to insert a device, such as an endotracheal tube, through the mouth or nose and into the trachea to establish and maintain a patent airway. In most cases, the endotracheal tube includes an inflatable cuff that surrounds the tube and that is inflated with air or another fluid to seal the tube within the trachea. Such a seal both ensures that air can reach the patient's lungs, for instance when positive pressure ventilation is performed, and that the lungs do not aspirate contents from the patient's stomach, for instance when the patient vomits while an individual is unconscious or only partially awake. Unfortunately, however, inflation of the endotracheal tube cuff can place excessive pressure on the patient's adjacent tissues, such as mucosa of the trachea, which can cause tissue damage.

Over the past two decades additional airway devices such as the laryngeal mask airway (LMA) have become a common airway management device used by the anesthesia and emergency responder community. Such devices do not have a cuff that is inflated within the trachea and further is not inserted into a trachea. Instead, an elliptical mask is provided at the end of a tube and is deployed within the pharynx to form a seal on top of the glottis. As a cuff is not inflated within the trachea, the type of damage to trachea that can be caused by endotracheal tubes is avoided. However, LMAs do not always form an adequate seal over the glottis and, therefore, may not actually secure the airway as would an endotracheal tube. This can, for example, be the case when the mask is not fully inflated or the inflated mask does not fit well the anatomy of the patient. Even if the mask fits well to the patient anatomy and creates a good seal, the pressure applied to the pharynx can cause tissue trauma and its related complications, such as throat pain, nausea and vomiting.

In addition, difficult trachea intubation is frequently encountered during emergency resuscitation outside of the hospital setting. LMA is a recommended alternative in case trachea intubation is difficult or impossible. However, the LMA may not be inserted appropriately by the medical professional and/or a gas jet generated from positive pressure ventilation may cause gas to enter the stomach.

Prior art examples of insertable tubes via the mouth are disclosed in published US Patent Application 2014/0216449 A1 (Los Angeles Biomedical Research Institute at Harbo-UCLA Medical Center), US Patent 3,841,319 (MICHAEL et al), published German Patent Application 3303582 A1 (ZAHLER et al), published International Patent Application WO 2007/061076 (NATIONAL UNIVERSITY CORPORATION OKAYAMA UNIVERSITY) and published US Patent Application 2007/163596 A1 (OLYMPUS MEDICAL SYSTEMS CORP). The document US 2014/0216449 A1, for example, discloses a fluid delivery and airway management device including a tubular member dimensioned for introducing a fluid into a trachea of a mammal, the tubular member having a proximal portion, a distal portion, and a middle portion between the proximal portion and the distal portion. The tubular member is dimensioned for positioning of the proximal portion in an oral cavity of a mammal, the middle portion in an oropharynx of the mammal and the distal portion in an esophagus of the mammal. An inflatable oral cavity balloon is positioned at the proximal portion and dimensioned to occlude the oral cavity. An inflatable esophageal balloon is positioned at the distal portion and dimensioned to occlude the esophagus. Apertures may be formed within the middle portion such that a fluid introduced into the tubular member is output through the apertures to a trachea. The device also includes a stabilizer. The stabilizer is positioned along a portion of tubular member positioned to anchor the gum thus stabilizes the device in the mouth.

In view of the above discussion, it can be appreciated that it would be desirable to have an alternative means for establishing a patent airway.

### Brief Description of the Drawings

The present disclosure may be better understood with reference to the following figures. Matching reference numerals designate corresponding parts throughout the figures, which are not necessarily drawn to scale.
Fig. 1 is a schematic view of a first embodiment of a pharyngeal tube, shown inserted in a patient's pharynx to establish a patent airway.
Fig. 2 is a front view of a patient intubated with the pharyngeal tube shown in Fig. 1.
Fig. 3 is a schematic view of a second embodiment of a pharyngeal tube.
Fig. 4 is a schematic view of a third embodiment of a pharyngeal tube.
Fig. 5 is a schematic view of a fourth embodiment of a pharyngeal tube.
Fig. 6 is a schematic view of a fifth embodiment of a pharyngeal tube.
Fig 7 is a schematic view of a sixth embodiment of a pharyngeal tube.
Fig 8 is a schematic view of a seventh embodiment of a pharyngeal tube.
Fig 9 is a schematic view of an eighth embodiment of a pharyngeal tube.

### Summary of the Invention

The invention relates to a pharyngeal tube for establishing an airway within a patient as defined in the claims. As described herein, the pharyngeal tube comprises: an elongated hollow tube including a proximal end and a distal end; a mouthpiece provided at the proximal end, wherein the mouthpiece is configured to provide an airtight seal at the mouth of the patient; and one or more lateral openings provided at or near the distal end of the hollow tube, the one or more lateral openings being formed in a lateral wall of the hollow tube; wherein a distal tip of the hollow tube is sealed so that air cannot escape the tube from its distal tip. The mouthpiece comprises an inner flange and an outer flange that are configured for placement on both the inside and outside of the patient's lips so as to form the airtight seal at the patient's mouth. The pharyngeal tube is configured to establish a patient airway without inflating a balloon or other sealing element within the trachea or pharynx by only sealing at the patient's mouth. In an embodiment, the inner flange is configured to be positioned between the patient's teeth and lips, and the outer flange configured to be positioned on the outside of the patient's lips. The hollow tube comprises multiple lateral openings or the hollow tube comprises a single lateral opening. In some embodiments, the pharyngeal tube further comprises a pressure relief valve configured to vent air from the hollow tube when pressure within the hollow tube exceeds a predetermined pressure threshold. In some embodiments, the hollow tube further includes an inner septum that divides the tube into first and second lumens that are connected at the distal end of the tube. In certain embodiments, the hollow tube further comprises an inner channel that that extends along the inner surface or in the wall of the tube. In some embodiments, the inner channel is configured to exist as a suction or injection catheter. In other embodiments, the inner channel is configured to exist as a catheter that can be used as a carbon dioxide or other gas sampling line. In some embodiments, the suction catheter is associated with a reservoir in which fluid removed from the hollow tube can be deposited. In some embodiments, the hollow tube has multiple inner channels. A first channel is for inspiratory gas flow and a second channel is for expiratory gas flow in order to reduce dead space of ventilation. In some embodiments, the first lumen is for inspiratory gas flow and the second lumen is for expiratory gas flow. In alternative embodiments the inner channel contains a gastric tube. In still other embodiments a gastric tube is attached and runs parallel to the pharyngeal tube.

The foregoing has outlined various features of the present disclosure in order that the detailed description that follows may be better understood and is not indicative of all possible embodiments of the invention. Additional features and advantages of the disclosure will be described hereinafter.

### Detailed Description

Referring to the drawings in general, not necessarily shown to scale, it will be understood that the illustrations are for the purpose of describing particular implementations of the disclosure and are not intended to be limiting thereto. While most of the terms used herein will be recognizable to those of ordinary skill in the art, it should be understood that when not explicitly defined, terms should be interpreted as adopting a meaning presently accepted by those of ordinary skill in the art.

It is to be understood that both the foregoing general description and the following detailed description are exemplary and explanatory only, and are not restrictive of the invention, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that comprise more than one unit unless specifically stated otherwise.

As described above, it would be desirable to have means for establishing a secure airway that minimize or avoid common complications, such as failure of establishing a patent airway, patient tissue trauma, or nausea with risk of aspiration associated with conventional devices. Disclosed herein are examples of such device and means. In one embodiment, a pharyngeal tube is used to establish a patent airway without inflating a balloon or other sealing element within the trachea or pharynx but only at the opening of the mouth. As used herein the term secure airway indicates a patent airway with an open pathway between a patient's lungs and the outside world. The pharyngeal tube comprises an elongated hollow tube that is configured for insertion into the pharynx via the mouth. At the proximal end of the tube are flanges that are configured for placement on both the inside and the outside of the patient's lips so as to form a seal at the patient's mouth. Near the distal end of the tube are one or more openings formed in the lateral walls of the tube that enable air to exit the tube in the lateral direction and enter the pharynx and trachea. As there is no opening provided at the distal tip of the tube, the air pressure is sufficient to maintain patency of the pharynx and no air is directed toward the esophagus, thereby minimizing or avoiding gastric insufflation. During expiration, the recoil of lung and chest wall generates a pressure gradient which allows the gas expired from lung to enter the side openings and exhaled through the pharyngeal tube.

In the following application, various specific embodiments are described. It is to be understood that those embodiments are example implementations of the disclosed inventions and that alternative embodiments are possible.

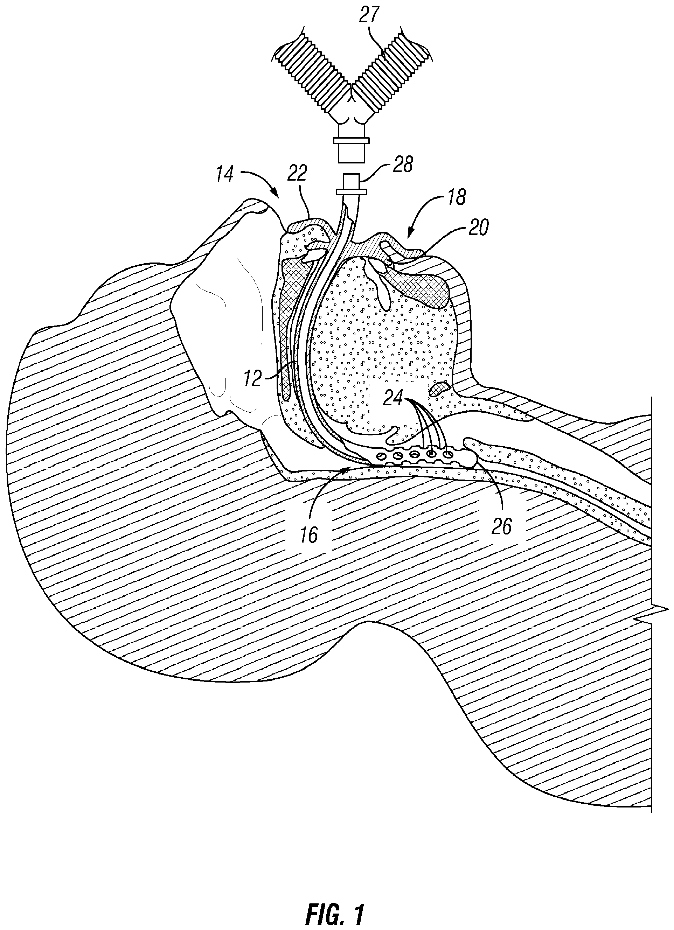

Fig. 1 illustrates an example embodiment of a pharyngeal tube 12, which is shown inserted into a patient so as to establish a patent airway. As is shown in the figure, the pharyngeal tube generally comprises an elongated hollow tube 12 having a proximal end 14 that is configured via an adapter to connect to the hose 27 of a ventilator (not shown) and a distal end 16 that is configured to be positioned within the oropharynx in proximity to the epiglottis and the trachea. The tube 12 is made of a suitable biocompatible material, such as polyvinyl chloride, and can either be semi-rigid or flexible. The various dimensions of the tube 12, including inner/outer diameter, length, curvature, etc., can be tailored so as to best suit the dimensions of patients of different sizes. Generally speaking, however, the tube 12 is long enough so that its distal end 16 can bypass the soft palate, tongue, and epiglottis, and the inner dimension (e.g., inner diameter) of the tube is sufficient to provide adequate air supply to the patient to maintain patency and facilitate adequate ventilation.

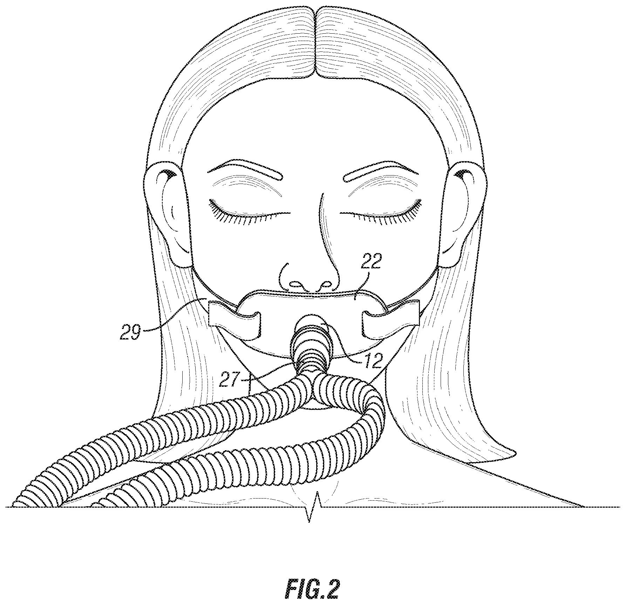

As is apparent in Fig. 1, a mouthpiece 18 is provided at the proximal end 14 of the tube 12. This mouthpiece 18 includes both an inner flange 20 configured to be positioned between the patient's lips and teeth or gums, and an outer flange 22 configured to be positioned against the outside of the patient's lips. With such a configuration, the mouthpiece 18 or inner/outer flanges 20, 22 can form an airtight seal at the patient's mouth, thereby preventing leakage of the pressurized air that is delivered using the pharyngeal tube 12. In some embodiments, the inner or outer flanges 20, 22 may be generally oblong-shaped. The inner flange 20 is generally smaller in size than the outer flange 22 so that it may be easily inserted in a patient's mouth and positioned between the lips and teeth or gums. The outer flange is shaped to be larger than a patient's mouth to allow an airtight seal to be formed. In some embodiments, the inner or outer flanges 20, 22 or mouthpiece 18 may be any suitable biocompatible material that is flexible or semi-flexible, which aids formation of the airtight seal. While the embodiments shown are generally planar, some embodiments of the inner or outer flanges 20, 22 or mouthpiece 18 may be shaped or contoured to aid formation of an airtight seal, such as, but not limited to a convex-shape. Analysis of a nonlimiting prototype's generally planar inner or outer flanges, comprised of silicone, illustrated that such flanges were capable of sustaining a desired airway pressure, such as of 40 cm H2O or greater. In some embodiments, an elastic strap may secure ends of the outer flange 22 or mouthpiece 18 (e.g. Fig. 2). Placement of the strap about the patient's head may further aid formation of an airtight seal.

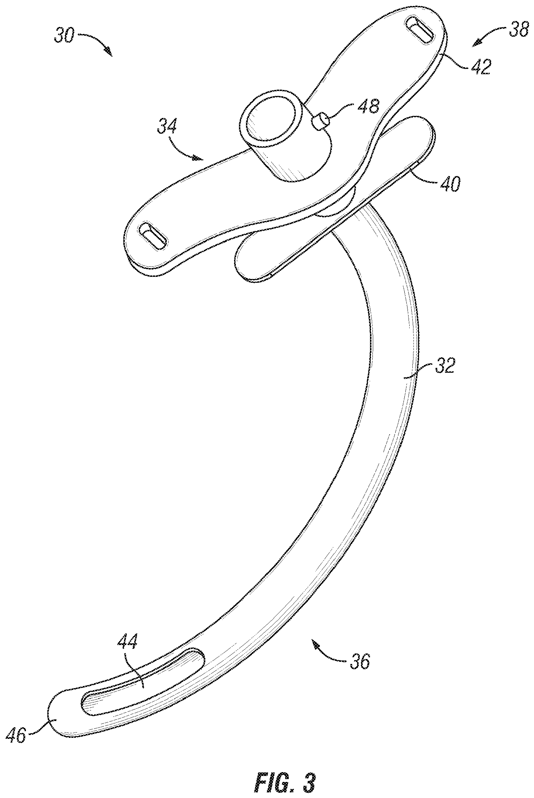

Provided at or near the distal end 16 of the tube 12 are one or more side openings 24 of sufficient size and shape to allow pressurized air to be delivered through the tube to the patient or ejected in reverse direction from the tube during expiration. Further, the tube 12 or openings 24 may also maintain patency of the pharynx and trachea enabling adequate ventilatory support. Because this design also allows positive end expiratory pressure (PEEP), for instance at 5-10 cm H2O, the pharynx is kept patent. If the patient is kept on spontaneous breathing, the negative pressure generated during inspiration and positive pressure generated by the recoiling of chest wall and lung create the pressure gradient through the side openings and allow air in during the inspiratory phase and out during the expiratory phase. If the patient is kept on mechanical ventilation, the inward gas flow is generated by the pressure gradient in the inspiratory phase and the reverse flow is generated by the recoiling of chest wall and lung as it occurs in the spontaneous breathing. The airtight seal of the device occurs at the mouth, therefore positioning of the device does not require a seal at the glottis. In the illustrated embodiment, these openings 24 may take the form of small circular shaped openings that are formed in the lateral wall of the tube 12, but it shall be understood by one of ordinary skill that any other shaped opening(s) may be utilized in other embodiments. Accordingly, the openings 24 may be referred to as lateral openings. Notably, while multiple openings 24 are shown in Fig. 1, in some embodiments a single, relatively large opening can be provided instead of multiple relatively small openings (e.g. Fig. 3). Such an embodiment allows for the insertion of a bronchoscope. This single large opening would be orientated to face anteriorly and a bronchoscope can be inserted through the large side opening. In addition, an endotracheal tube can be inserted using the bronchoscope as the guide wire. The single opening can be arranged at different angles relative to the central axis of the tube 12 and are in no way limited to the particular arrangements or orientations shown in the figures.

Although one or more lateral openings 24 are provided near the distal end 16 of the tube 12, it is noted that the distal tip 26 of the tube is sealed (i.e., comprises no openings) so that air can only escape from the tube in lateral directions.

With further reference to Fig. 1, the proximal tip of the tube 12 can comprise a suitable connector 28 that is configured to connect to the ventilator tube.

To use the pharyngeal tube 12, it is passed through the patient's mouth, soft palate, tongue, and epiglottis until the distal end 16 is positioned in the pharynx adjacent to the trachea. In some embodiments, the distal end 16 may reach the proximal portion of esophagus or possibly enter trachea through glottis. The patient's lips are positioned between the inner and outer flanges 20, 22 so as to form a seal. For example, the inner flange 20 is positioned between the patient's lips and teeth or gums, and an outer flange 22 is positioned against the outside of the patient's lips. Next, the ventilator tube can be connected to the connector 28 located at the proximal tip of the tube 12. When this positioning is performed, the tube 12 will be positioned in similar manner to that shown in Fig. 1.

Fig. 2 shows the intubated patient in front view. Once the tube 12 has been so positioned, pressurized air generated by the ventilator can be delivered to the tube 12. This air flows through the tube 12 and is ultimately ejected from the lateral openings 24. The pressure provided by this ejection maintains the pharynx orientation such that the air is delivered to the patient's lungs. Also shown in Fig. 2 is a ventilator tube 27 connectable to tube 12, and a strap 29 that secures the mouthpiece 18 or outer flange 22 to the patient's head. Such a tube 12 can serve as the conduit for spontaneous ventilation as well, if the patient's own ventilatory effort can generate adequate pressure gradient through the tube 12.

As discussed previously, the pharyngeal tube need not include multiple openings. For example, the tube can instead comprise a single, relatively large opening. Fig. 3 illustrates an example of such an embodiment. As shown in this figure, the pharyngeal tube 30 is similar in many ways to the pharyngeal tube 12 shown in Figs. 1 and 2. Therefore, the pharyngeal tube 30 generally includes an elongated hollow tube 32 having a proximal end 34 and a distal end 36. A mouthpiece 38 is provided at the proximal end 34 of the tube 32 and includes an inner flange 40 and an outer flange 42. In this embodiment, however, a single, relatively large lateral opening 44 is provided at or near the distal end 36 of the tube 32. In the illustrated embodiment, this opening 44 is positioned on the inner side of a curve traced by the tube 32 such that, when inserted, it will face the trachea. As a nonlimiting example, with this relatively larger lateral opening 44 in comparison to other embodiments, a bronchoscope (not shown) can be passed through the tube 32 and out through the opening into the trachea. In addition, the large lateral opening 44 can be used to perform tracheal intubation. As with the tube 12, the distal tip 46 of the tube 32 is sealed. In the illustrated embodiment, the pharyngeal tube 30 also includes a pressure relief valve 48 that is configured to vent air from the tube 32 when the pressure within the tube exceeds a predetermined threshold, for example, 25 cm of water.

It shall be understood that the exemplary embodiments discussed above may incorporate other features from further embodiments discussed herein or vice versa. For example, the pressure relief valve 48 may be included in the initial embodiment of Figs. 1-2 or any of the other embodiments discussed herein. Further, it shall be recognized by one of ordinary skill that various features discussed further herein, such as but not limited to an inner septum, inner channel, integrated of separate gastric channel, etc., are not limited to the embodiments shown and may be incorporate or merged with other embodiments.

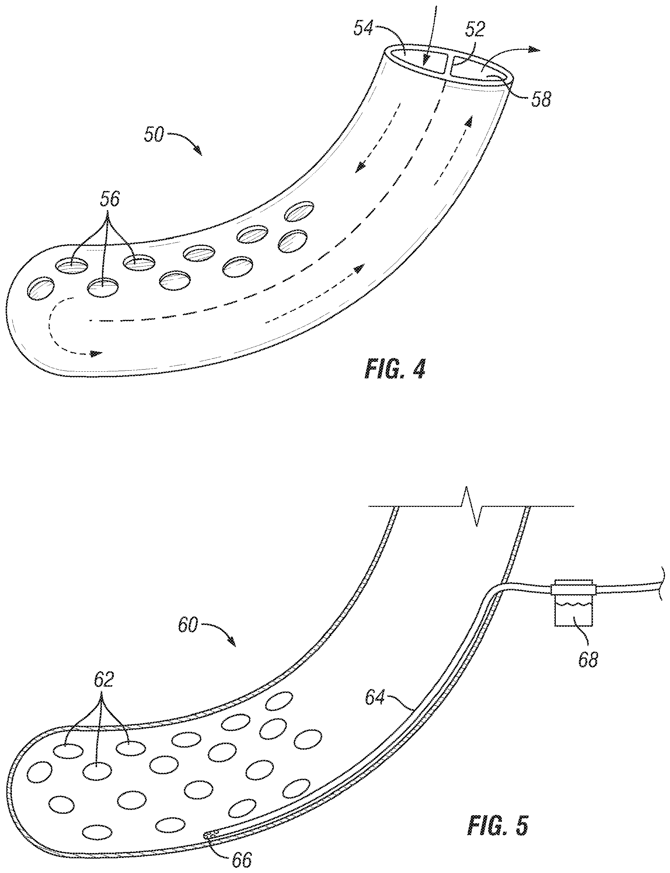

Fig. 4 illustrates a further embodiment for the elongated tube that can be used in a pharyngeal tube of the type described above. Illustration of the proximal end of the elongated tube, mouthpiece, and other components are omitted for clarity, but it shall be understood they may be present. As shown in Fig. 4, the tube 50 can comprise an inner septum 52 that separates the inside of the tube into two lumens. In this embodiment, the septum does not run the full length of the tube such that the two lumens join each other at the distal end of the tube. Pressurized air can be delivered to a first lumen 54 defined by the septum 52 and ejected from one or more lateral opening(s) 56. If any fluid enters and collects in the bottom of the tube 50, it can be removed via a second lumen 58 defined by the septum 52, which can be connected to a suction source (not shown). With this functionality, the gas enters via the first lumen and exhaled via the second lumen in order to reduce dead space ventilation and improve breathing efficiency.

Fig. 5 illustrates yet another embodiment in which a separate channel is built into the elongated tube that can be used in a pharyngeal tube of the type described herein. Illustration of the proximal end of the elongated tube, mouthpiece, and other components are omitted for clarity, but it shall be understood they may be present. As shown in Fig. 5, the tube 60 includes one or more lateral opening(s) 62 just like the tube 50. In addition, however, the tube 60 includes a small inner channel 64 that extends along at least a portion of the inner surface of the tube. A small diameter suction catheter 66 can be passed through the channel 64 in order to remove any fluid that has collected within the tube 60. This fluid can be deposited in a reservoir 68 associated with the catheter 66. Channel 64 can also be used to sample carbon dioxide gas for measurement of end-tidal carbon dioxide concentration. Should gastric content enter the pharyngeal cavity, because the pharyngeal tube lies below the glottis when the patient is in a supine position, gravity will assist in sustaining the liquid in the lumen of the pharyngeal tube where the liquid could be removed by suction via the CO2 sampling line before it can be aspirated into the lungs. Such liquid in the CO2 sampling line of the pharyngeal tube would alert the medical professional and help manage aspiration. If it is used for sampling carbon dioxide, chamber 68 can serve as a reservoir to trap secretions emanating from the patient. In some embodiments the catheter 66 can be a built-in or integrated, instead of a channel for insertion of a catheter.

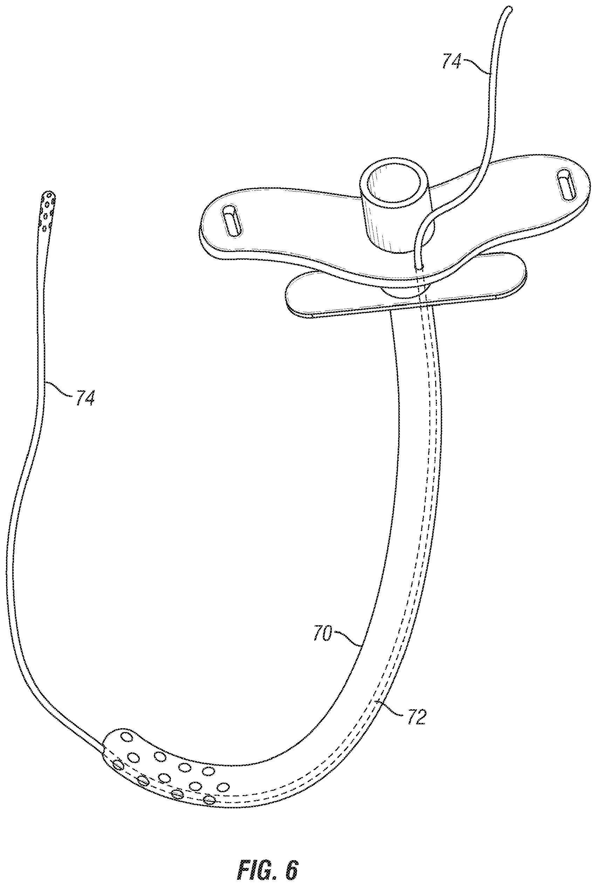

Fig. 6 illustrates yet another embodiment of the pharyngeal tube 70 that has a separate channel 72 for insertion of a gastric tube 74 as shown. This channel 72 is used for insertion of a gastric tube 74 that enters the esophagus for positioning distally. The proximal end of the channel 72 is adjacent to the proximal opening of the pharyngeal tube 70, the channel runs through at least a portion of the pharyngeal tube, and the distal end of the channel is located at or near the distal end of the pharyngeal tube. A gastric tube 74 inserted through the channel 72 enters esophagus and then stomach. When ventilation occurs through a pharyngeal tube, gastric content including insufflated air can be suctioned out using negative pressure applied to the proximal end of the gastric tube 74. In some embodiments the separate channel 72 allows the insertion of alternative devices, such as, but not limited to endoscopic or other devices.

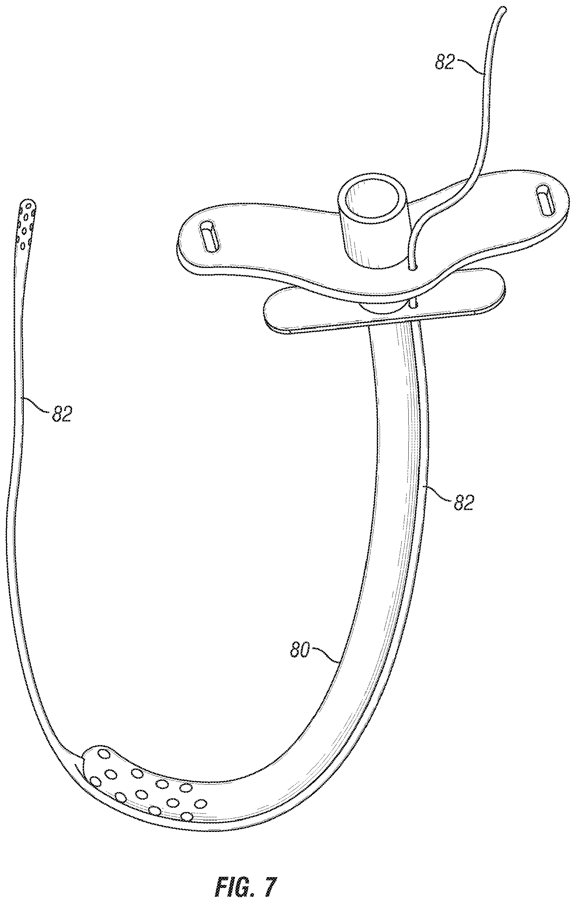

Fig. 7 illustrates an embodiment of the pharyngeal tube 80 in which the gastric tube 82 is separated from and parallel to the pharyngeal tube. The gastric tube is inserted through the esophagus and then into the stomach. When ventilation occurs through a pharyngeal tube, gastric content including insufflated air can be suctioned out using negative pressure applied to the proximal end of the gastric tube.

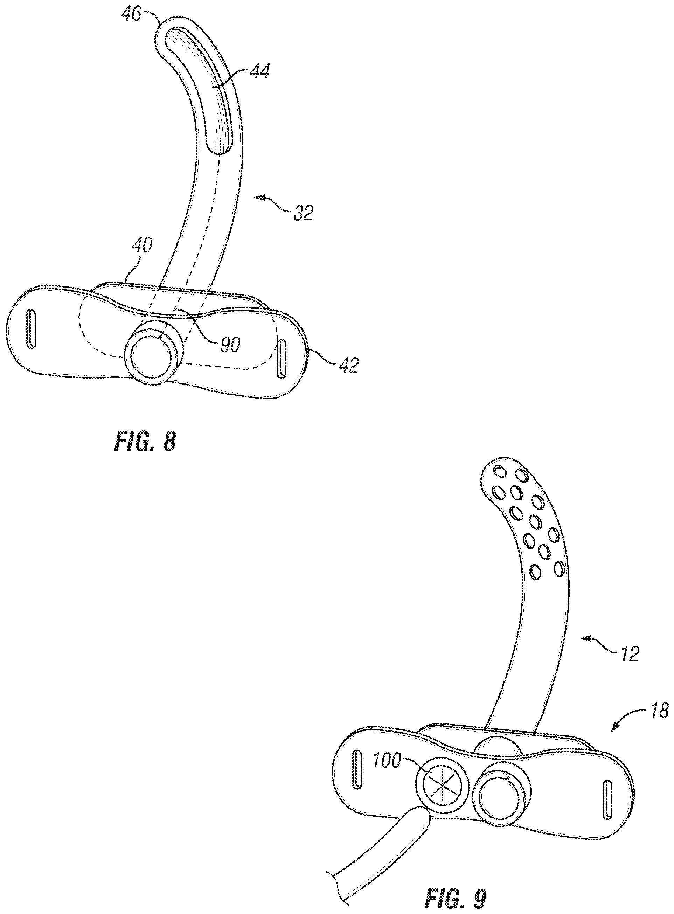

Fig. 8 illustrates an embodiment in which the tube comprises a single, relatively large opening 44 similar to the embodiment of Fig. 3. Various components sharing the same numbering as Fig. 3 are also present (see prior discussion of Fig. 3 for further detail). Starting near the proximal end of the pharyngeal tube 32 are perforations extending lengthwise 90 along the wall of the tube all the way to the opening 44 (Fig. 8).

Fig. 9 illustrates an embodiment of the pharyngeal tube which encompasses the pharyngeal tube 12, and further comprises an additional port 100 in the mouthpiece 18 that may allow an endoscope or the like to be inserted through the mouthpiece. The port 100 may be positioned on the mouthpiece adjacent to the opening for tube 12. These ports may be fitted with any suitable one-way valve, by way of example but not limitation, a check valve, multiple flexible flap or membrane valve, for example, a duckbill valve. This allows the maintenance of a patent airway using positive pressure ventilation while also using the endoscope. This configuration allows the endoscopy to be performed with a patient under deep sedation and without tracheal intubation.

Embodiments described herein are included to demonstrate particular aspects of the present disclosure. It should be appreciated by those of skill in the art that the embodiments described herein merely represent exemplary embodiments of the disclosure. Those of ordinary skill in the art should, in light of the present disclosure, appreciate that many changes can be made in the specific embodiments described, including various combinations of the different elements, components, steps, features, or the like of the embodiments described, and still obtain a like or similar result without departing from the scope of the present disclosure. From the foregoing description, one of ordinary skill in the art can easily ascertain the essential characteristics of this disclosure, and without departing from the scope thereof, can make various changes and modifications to adapt the disclosure to various usages and conditions. The embodiments described hereinabove are meant to be illustrative only and should not be taken as limiting of the scope of the disclosure. The scope of the present invention is defined by the following appended claims.

## Claims

1. A pharyngeal tube comprising:
an elongated hollow tube (12, 32) including a proximal end (14, 34) and a distal end (16, 36) for establishing a patent airway for a patient;
a mouthpiece (18, 38) provided at the proximal end, wherein the mouthpiece is configured to provide an airtight seal at the mouth of the patient, wherein the mouthpiece comprises an inner flange (20, 40) and an outer flange (22, 42) that are configured for placement on both the inside and outside of the patient's lips so as to form the airtight seal at the patient's mouth; and
one or more lateral openings (24, 44, 56) provided at or near the distal end (16, 36) of the hollow tube, wherein the one or more lateral openings are formed in a lateral wall of the hollow tube, and a distal tip (26, 46) of the hollow tube is sealed so that air cannot escape the tube from its distal tip,
wherein the pharyngeal tube is configured to establish a patent airway without inflating a balloon or other sealing element within the trachea or pharynx but only sealingat the patient's mouth.

2. The pharyngeal tube of claim 1, wherein the inner flange is configured to be positioned between teeth and lips of the patient, and the outer flange is configured to be positioned on an outside of the lips of the patient.

3. The pharyngeal tube of claim 2, wherein the inner flange and outer flange are oblong-shaped, and the outer flange is larger than a mouth of the patient.

4. The pharyngeal tube of claim 2, wherein the inner flange, outer flange, or mouthpiece are formed from a biocompatible material that is flexible or semi-flexible.

5. The pharyngeal tube of claim 1, wherein, when the hollow tube comprises a single, relatively large lateral opening (44), the hollow tube further comprises perforations (90) extending lengthwise along a wall of the tube from near the proximal end of the hollow tube all the way to the lateral opening (44).

6. The pharyngeal tube of claim 1, wherein the hollow tube further comprises an inner septum (52) that divides the tube into first and second lumens (54, 58) that are connected at the distal end of the tube.

7. The pharyngeal tube of claim 6, wherein the first lumen is for inspiratory gas flow, and the second lumen is for expiratory gas flow.

8. The pharyngeal tube of claim 1, wherein the hollow tube further comprises an inner channel (64) that extends along the inner surface of the tube.

9. The pharyngeal tube of claim 8, wherein the inner channel is configured to receive a suction catheter or a catheter that can be used as a carbon dioxide or gas sampling line or a gastric tube.

10. The pharyngeal tube of claim 1, wherein the pharyngeal tube has an attached gastric tube (82) that runs parallel.

11. The pharyngeal tube of claim 1, further comprising a pressure relief valve (48) configured to vent air from the hollow tube when pressure within the hollow tube exceeds a predetermined pressure threshold.

12. The pharyngeal tube of claim 1, wherein the mouthpiece further comprises an additional port (100) capable of allowing an endoscope to be inserted.

## Patentansprüche

1. Ein Pharyngealtubus, beinhaltend:
einen länglichen, hohlen Tubus (12, 32), umfassend ein proximales Ende (14, 34) und ein distales Ende (16, 36), zum Etablieren eines freien Atemwegs für einen Patienten; ein Mundstück (18, 38), das an dem proximalen Ende bereitgestellt ist, wobei das Mundstück konfiguriert ist, um eine luftdichte Abdichtung an dem Mund des Patienten bereitzustellen, wobei das Mundstück einen inneren Flansch (20, 40) und einen äußeren Flansch (22, 42) beinhaltet, die zur Platzierung an sowohl der Innenseite als auch der Außenseite der Lippen des Patienten konfiguriert sind, um die luftdichte Abdichtung an dem Mund des Patienten zu bilden; und
eine oder mehrere seitliche Öffnungen (24, 44, 56), die an oder nahe dem distalen Ende (16, 36) des hohlen Tubus bereitgestellt sind, wobei die eine oder die mehreren Öffnungen in einer seitlichen Wand des hohlen Tubus gebildet sind und eine distale Spitze (26, 46) des hohlen Tubus so abgedichtet ist, dass Luft dem Tubus nicht aus seiner distalen Spitze entweichen kann,
wobei der Pharyngealtubus konfiguriert ist, um einen freien Atemweg ohne Aufblasen eines Ballons oder eines anderen abdichtenden Elements innerhalb der Trachea oder des Pharynx, sondern nur durch Abdichten an dem Mund des Patienten zu etablieren.

2. Pharyngealtubus gemäß Anspruch 1, wobei der innere Flansch konfiguriert ist, um zwischen Zähnen und Lippen des Patienten positioniert zu werden, und der äußere Flansch konfiguriert ist, um an einer Außenseite der Lippen des Patienten positioniert zu werden.

3. Pharyngealtubus gemäß Anspruch 2, wobei der innere Flansch und der äußere Flansch langgestreckt gestaltet sind und der äußere Flansch größer als ein Mund des Patienten ist.

4. Pharyngealtubus gemäß Anspruch 2, wobei der innere Flansch, der äußere Flansch oder das Mundstück aus einem biokompatiblen Material gebildet sind, das flexibel oder halbflexibel ist.

5. Pharyngealtubus gemäß Anspruch 1, wobei, wenn der hohle Tubus eine einzelne, relativ große seitliche Öffnung (44) beinhaltet, der hohle Tubus ferner Perforationen (90) beinhaltet, die sich der Länge nach entlang einer Wand des Tubus von nahe dem proximalen Ende des hohlen Tubus den ganzen Weg bis zu der seitlichen Öffnung (44) erstrecken.

6. Pharyngealtubus gemäß Anspruch 1, wobei der hohle Tubus ferner ein inneres Septum (52) beinhaltet, das den Tubus in ein erstes und ein zweites Lumen (54, 58) teilt, die an dem distalen Ende des Tubus verbunden sind.

7. Pharyngealtubus gemäß Anspruch 6, wobei das erste Lumen für inspiratorischen Gasfluss ist und das zweite Lumen für expiratorischen Gasfluss ist.

8. Pharyngealtubus gemäß Anspruch 1, wobei der hohle Tubus ferner einen inneren Kanal (64) beinhaltet, der sich entlang der inneren Oberfläche des Tubus erstreckt.

9. Pharyngealtubus gemäß Anspruch 8, wobei der innere Kanal konfiguriert ist, um einen Saugkatheter oder einen Katheter, der als eine Kohlendioxid- oder Gasprobenahmeleitung verwendet werden kann, oder einen Magentubus aufzunehmen.

10. Pharyngealtubus gemäß Anspruch 1, wobei der Pharyngealtubus einen befestigten Magentubus (82) aufweist, der parallel verläuft.

11. Pharyngealtubus gemäß Anspruch 1, ferner beinhaltend ein
Druckentlastungsventil (48), das konfiguriert ist, um Luft aus dem hohlen Tubus abzulassen, wenn der Druck innerhalb des hohlen Tubus einen vorher festgelegten Druckschwellenwert übersteigt.

12. Pharyngealtubus gemäß Anspruch 1, wobei das Mundstück ferner einen zusätzlichen Anschluss (100) beinhaltet, der geeignet ist, es zu ermöglichen, dass ein Endoskop eingesetzt wird.

## Revendications

1. Un tube pharyngé comprenant :
un tube creux allongé (12, 32) incluant une extrémité proximale (14, 34) et une extrémité distale (16, 36) servant à établir des voies respiratoires dégagées pour un patient ;
un embout buccal (18, 38) fourni au niveau de l'extrémité proximale, l'embout buccal étant configuré pour fournir un joint hermétique au niveau de la bouche du patient, l'embout buccal comprenant une collerette interne (20, 40) et une collerette externe (22, 42) qui sont configurées pour un placement tant sur l'intérieur que sur l'extérieur des lèvres du patient de manière à conformer le joint hermétique au niveau de la bouche du patient ; et
une ou plusieurs ouvertures latérales (24, 44, 56) fournies au niveau ou à proximité de l'extrémité distale (16, 36) du tube creux, les une ou plusieurs ouvertures latérales étant conformées dans une paroi latérale du tube creux, et un bout distal (26, 46) du tube creux étant scellé hermétiquement de sorte que de l'air ne puisse pas s'échapper du tube depuis son bout distal,
le tube pharyngé étant configuré pour établir des voies respiratoires dégagées sans gonfler de ballonnet ni un autre élément de scellement à l'intérieur de la trachée ou du pharynx mais uniquement en scellant hermétiquement au niveau de la bouche du patient.

2. Le tube pharyngé de la revendication 1, dans lequel la collerette interne est configurée pour être positionnée entre des dents et les lèvres du patient, et la collerette externe est configurée pour être positionnée sur un extérieur des lèvres du patient.

3. Le tube pharyngé de la revendication 2, dans lequel la collerette interne et la collerette externe sont de forme oblongue, et la collerette externe est plus grande que la bouche du patient.

4. Le tube pharyngé de la revendication 2, dans lequel la collerette interne, la collerette externe, ou l'embout buccal sont conformés à partir d'un matériau biocompatible qui est flexible ou semi-flexible.

5. Le tube pharyngé de la revendication 1, dans lequel, lorsque le tube creux comprend une seule ouverture latérale (44) relativement grande, le tube creux comprend en outre des perforations (90) s'étendant dans le sens de la longueur le long d'une paroi du tube depuis près de l'extrémité proximale du tube creux complètement jusqu'à l'ouverture latérale (44).

6. Le tube pharyngé de la revendication 1, dans lequel le tube creux comprend en outre une cloison interne (52) qui divise le tube en des première et deuxième lumières (54, 58) qui sont connectées au niveau de l'extrémité distale du tube.

7. Le tube pharyngé de la revendication 6, dans lequel la première lumière sert à un flux de gaz d'inspiration, et la deuxième lumière sert à un flux de gaz d'expiration.

8. Le tube pharyngé de la revendication 1, dans lequel le tube creux comprend en outre un canal interne (64) qui s'étend le long de la surface interne du tube.

9. Le tube pharyngé de la revendication 8, dans lequel le canal interne est configuré pour recevoir un cathéter d'aspiration ou un cathéter qui peut être utilisé comme une conduite de prélèvement de dioxyde de carbone ou de gaz ou un tube gastrique.

10. Le tube pharyngé de la revendication 1, le tube pharyngé ayant un tube gastrique (82) lui étant attaché qui lui est parallèle.

11. Le tube pharyngé de la revendication 1, comprenant en outre une soupape de surpression (48) configurée pour évacuer de l'air du tube creux lorsqu'une pression à l'intérieur du tube creux dépasse une pression seuil prédéterminée.

12. Le tube pharyngé de la revendication 1, dans lequel l'embout buccal comprend en outre un orifice supplémentaire (100) à même de permettre qu'un endoscope soit inséré.
